# EUROPEAN PATENT APPLICATION

(11) **EP 4 663 196 A1**
(43) Date of publication of application: **17.12.2025**
(21) Application number: 24182102.4
(22) Date of filing: 13.06.2024
(51) Int. Cl.: A61K 35/741, A61P 31/04

(54) **DECOLONIZATION OF PATHOGENIC ENTEROBACTERIA, SUCH AS E. COLI AND/OR KLEBSIELLA PNEUMONIAE, FROM THE GUT USING STRAINS OF E. COLI**

(71) Applicant: Helmholtz-Zentrum für Infektionsforschung GmbH, 38124 Braunschweig (DE); Otto-von-Guericke-Universität Magdeburg Körperschaft des Öffentlichen Rechts, 39106 Magdeburg (DE)
(72) Inventor: STROWIG, Till, 38124 Braunschweig (DE); OSBELT-BLOCK, Lisa, 38124 Braunschweig (DE); WENDE, Marie, 39106 Magdeburg (DE)
(74) Representative: Krauss, Jan

(57) **Abstract**

The present invention relates to probiotic bacteria of the species *E. coli,* in particular in combination with bacteria of the species *Klebsiella oxytoca,* that are used for a decolonization of pathogenic and/or multi drug resistant (MDR) *Enterobacteria,* such as pathogenic *E. coli* and/or *Klebsiella pneumoniae (K. pneumoniae), Enterococci* and/or *Acinetobacter,* from the gut of a subject. The decolonization can both be therapeutic, i.e. after colonization of the gut by the pathogenic and/or multi-resistant pathogen(s), or as a preventive measure before a re-colonization of the gut, as required after antibiotic treatment or treatment-induced dysbiosis.

## Description

The present invention relates to probiotic bacteria of the species *E. coli,* in particular in combination with bacteria of the species *Klebsiella oxytoca,* that are used for a decolonization of pathogenic and/or multi drug resistant (MDR) *Enterobacteria,* such as pathogenic *E. coli* and/or *Klebsiella pneumoniae (K. pneumoniae), Enterococci* and/or *Acinetobacter,* from the gut of a subject. The decolonization can both be therapeutic, i.e. after colonization of the gut by the pathogenic and/or multi-resistant pathogen(s), or as a preventive measure before a re-colonization of the gut, as required after antibiotic treatment or treatment-induced dysbiosis.

### Background of the invention

Multi-drug resistance in the family of *Enterobacteriaceae* is one of the greatest health associated problems worldwide (WHO. United Nations meeting on antimicrobial resistance. Bull World Health Organ. 2016;94(9):638-639. doi:10.2471/blt.16.020916). Especially the incidence of infections as well as the mortality rates linked to carbapenem-resistantK. *pneumoniae* is constantly increasing and has reached alarming rates in Europe (Cassini, Alessandro et al (2018). Attributable deaths and disability-adjusted life-years caused by infections with antibiotic-resistant bacteria in the EU and the European Economic Area in 2015: a population-level modelling analysis. The Lancet Infectious Diseases. 19. 10.1016/S1473-3099(18)30605-4).

*Klebsiella pneumoniae* (KLP), a Gram-negative bacterium belonging to the family of Enterobacteriaceae, is a common cause of antimicrobial-resistant opportunistic infections in hospitalized patients. KLP can colonize in the human gastrointestinal tract, especially in patients with inflammatory bowel diseases, activate caspase-11 inflammasomes, and contribute to intestinal inflammation (Zhang Q, Su X, Zhang C, Chen W, Wang Y, Yang X, Liu D, Zhang Y, Yang R. Klebsiella pneumoniae Induces Inflammatory Bowel Disease Through Caspase-11-Mediated IL18 in the Gut Epithelial Cells. Cell Mol Gastroenterol Hepatol. 2023;15(3):613-632. doi: 10.1016/j.jcmgh.2022.11.005. Epub 2022 Nov 25. PMID: 36436756; PMCID: PMC9871440.).

Several mechanisms of colonization resistance against intestinal pathogens have been described, including competition for nutrients, short chain fatty acid (SCFA)-dependent inhibition of virulence and replication, and direct antagonism through bacteriocin production or Type VI Secretion System-mediated killing.

In healthcare settings, antibiotic-mediated disruption of the microbiota can be associated with enteric expansion of members of the *Enterobacteriaceae* family, including *E. coli* and *Klebsiella pneumoniae,* as well as vancomycin-resistant *Enterococci* and *Clostridium difficile* (Kim S., Covington A., and Pamer E.G. 2017. The intestinal microbiota: Antibiotics, colonization resistance, and enteric pathogens. Immunol. Rev. 279:90-105. 10.1111/imr.12563). While C. *difficile* causes gastroenteritis, hospital-associated *Enterobacteriaceae* and vancomycin-resistant *Enterococci* strains often expand in the gut without triggering overt inflammatory responses. In these instances, the primary clinical concern is that expansion of these species in the gut increases the risk for subsequent development of a bloodstream infection (BSI) in vulnerable patient populations. For example, allogeneic hematopoietic stem cell transplantation (allo-HCT) is an effective but highly immunocompromising treatment for some forms of cancer. The high incidence and risk of bacterial infections in patients undergoing allo-HCT necessitates administration of prophylactic and empiric antibiotics, leading to destruction of the normal microbiome. In this patient population, enteric domination with *Enterobacteriaceae* leads to a significant increase in the risk of developing a BSI. Furthermore, an increasing proportion of clinical isolates of *Enterobacteriaceae* are resistant to a wide range of antibiotics, including strains that produce extended-spectrum-β-lactamases or carbapenemases. Accordingly, BSI with these highly antibiotic-resistant strains are increasingly challenging to treat. *Klebsiella pneumoniae, Escherichia coli,* and other members of the *Enterobacteriaceae* family are common human pathogens that have acquired broad antibiotic resistance, rendering infection by some strains virtually untreatable. *Enterobacteriaceae* are intestinal residents, but generally represent <1% of the adult colonic microbiota.

Sorbara et al. (in: Sorbara MT, et al. Inhibiting antibiotic-resistant Enterobacteriaceae by microbiota-mediated intracellular acidification. J Exp Med. 2019 Jan 7;216(1):84-98. doi: 10.1084/jem.20181639. Epub 2018 Dec 18) demonstrate that an antibiotic-naive microbiota suppresses growth of antibiotic-resistant clinical isolates of *Klebsiella pneumoniae, Escherichia coli,* and *Proteus mirabilis* by acidifying the proximal colon and triggering short chain fatty acid (SCFA)-mediated intracellular acidification. High concentrations of SCFAs and the acidic environment counter the competitive edge that O₂ and NO₃ respiration confer upon *Enterobacteriaceae* during expansion. Reestablishment of a microbiota that produces SCFAs enhances clearance of *Klebsiella pneumoniae, Escherichia coli,* and *Proteus mirabilis* from the intestinal lumen and represents a potential therapeutic approach to enhance clearance of antibiotic-resistant pathogens.

Fecal microbiota transplant (FMT), also known as a stool transplant, is the process of transferring fecal bacteria and other microbes from a healthy individual into another individual. FMT involves restoration of the colonic microflora by introducing healthy bacterial flora through infusion of stool via colonoscopy, enema, orogastric tube, or by mouth in the form of a capsule containing feces from a healthy donor, which in some cases is freeze-dried. FMT, for example, is an effective treatment for *Clostridioides difficile* infection (CDI) and may be more effective than vancomycin treatment. Side effects may include a risk of infections; therefore the donor should be screened.

With CDI becoming more common, FMT is gaining increasing prominence, with some experts calling for it to become the first-line therapy for CDI. FMT has been used experimentally to treat other gastrointestinal diseases, including colitis, constipation, irritable bowel syndrome, and neurological conditions, such as multiple sclerosis and Parkinson's. In the United States, human feces has been regulated as an experimental drug since 2013.

Caballero et al. (in: Silvia Caballero, et al., Cooperating Commensals Restore Colonization Resistance to Vancomycin-Resistant Enterococcus faecium, Cell Host & Microbe, Vol 21, 5, 2017, pp. 592-602.e4, https://doi.org/10.1016/j.chom.2017.04.002.2017) demonstrate that a precisely defined consortium of commensal bacteria containing the *Clostridium* cluster XIVa species *Blautia producta* and *Clostridium bolteae* restores colonization resistance against VRE and clears VRE from the intestines of mice.

O. Ljungquist et al. (in: Probiotics for intestinal decolonization of ESBL-producing Enterobacteriaceae: a randomized, placebo-controlled clinical trial. Clinical Microbiology and Infection 26 (2020) 456e462 https://doi.org/10.1016/j.cmi.2019.08.019) disclose a randomized, placebo-controlled, single-blinded clinical superiority trial, where the probiotic Vivomixx^{®}, a mixture of 8 different living bacterial strains or placebo was given to adult outpatients intestinally colonized for at least 3 months with extended spectrum b-lactamase (ESBL)-producing *Enterobacteriaceae* (EPE). Successful EPE eradication was observed only in very few individuals.

Lagrafeuille et al. (in: Lagrafeuille R, et al. Opposing effect of Lactobacillus on in vitro Klebsiella pneumoniae in biofilm and in an in vivo intestinal colonization model. Benef Microbes. 2018 Jan 29;9(1):87-100. doi: 10.3920/BM2017.0002. Epub 2017 Oct 12. PMID: 29022382) analyze the anti-biofilm activity of 140 neutralized *Lactobacillus* supernatants was assessed against *K. pneumoniae.* Among the 13 strains whose supernatant significantly impaired biofilm formation, *Lactobacillus plantarum* CIRM653 was selected because it was also able to impair *K. pneumoniae* preformed biofilm, independently of a bactericidal effect.

EP2040724B1 discloses a biotherapeutic composition comprising a pharmaceutically effective amount of a probiotic *Escherichia coli* strain, a pharmaceutically effective amount of an anaerobic bacteria antibiotic to which said *Escherichia coli* strain is resistant, and a pharmaceutically acceptable carrier, wherein said antibiotic is metronidazole, and wherein said *Escherichia coli* strain and said antibiotic act in synergy.

WO 2021/030198A1 discloses a composition comprising (1) one or more purified bacterial strains belonging to the phylum Firmicutes or Bacteroidetes and (2) one or more purified bacterial strains selected from the group consisting of an Escherichia species and a Fusobacterium species. Also disclosed are methods of suppressing colonization of the intestine of a subject with oral microbiome bacteria.

US7018629 B2 relates to pharmaceutical compositions of probiotic *E. coli* strains and uses thereof for treating inflammatory bowel disease.

The search for alternative intervention strategies new therapeutics is therefore urgently required. Other objects and advantages will become apparent to the person of skill when studying the present description of the present invention.

In a first aspect of the present invention, the above object is solved by a bacterium selected from the group of consisting of at least one of *E. coli* strains MR010, MK031, MR043, MR045, MR102, MR134, MR158, MK241, MK249, MK267, MK270, MK289, MR107, MR050, MK254, MR174, MR084, MK251, MK062, MR097, MK270, MR138, MK262, and MK179 for use in the prevention and/or treatment of pathogenic and/or antibiotic resistant *Enterobacteriaceae,* such *as E. coli* and/or *Klebsiella pneumoniae,* in particular multi-resistant (MDR) *Enterobacteriaceae, Enterococci* and/or *Acinetobacter.*

Preferred is the bacterium for use according to the present invention, wherein said use is in combination with at least one bacterium selected from the group of *Klebsiella michiganensis and*/*or Klebsiella oxytoca,* in particular from the strain MK01, MR08, MR050 and species related to these strains, and wherein preferably the combination shows a synergistic effect in the prevention and/or treatment. "Combination" shall mean both a mixture of the strains or separate preparations that are given jointly or sequentially to the patient, in order to act together, in particular synergistically.

Preferred is the bacterium for use according to the present invention, wherein said prevention and/or treatment is through decolonization of said *Enterobacteriaceae, Enterococci* and/or *Acinetobacter.*

The present invention provides an alternative treatment strategy that allows for an effective decolonization of multi-resistant bacteria and/or can prevent a colonization with such bacteria.

There is currently no medically recommended treatment strategy for intestinal decolonization with pathogenic and/or multi-resistantK. *pneumoniae, Enterococci* and/or *Acinetobacter,* and *E*. *coli* strains. Reserve antibiotics are currently used for this purpose, but they further worsen the problem of resistance development and also have strong effects on the natural protective effect of the microbiome. Alternative approaches such as fecal microbiota transplantation (FMT), used to treat *Clostridioides difficile* infections, have not been shown to be effective in treating MDR enterobacteria. In addition, it has already been shown that probiotics such as bifidobacteria, lactobacilli and *E. coli* Nissle can have a positive effect on the host. However, there are currently no probiotics available on the market that could displace MDR *E. coli* from the intestine.

FMT is an experimental therapy approach that is only used as a last resort because there are serious safety concerns with this form of therapy. Since a complex undefined bacterial community is transmitted, MDR bacteria can also be transmitted. The administration of probiotics has not yet been proven to have any effect on colonization with MDR enterobacteria. Furthermore, recent studies have shown that the probiotic strain *E. coli* Nissle produces the toxin colibactin.

In a second aspect of the present invention, the above object is solved by the bacterium for use as above in reestablishing colonization resistance after antibiotic treatment or treatment-induced dysbiosis in the microbiome of an animal, in particular a mammalian subj ect.

A third aspect of the present invention then relates to a method for preventing and/or treating pathogenic and/or antibiotic resistant *Enterobacteriaceae,* such as *E. coli* and/or *Klebsiella pneumoniae,* in particular multi-resistant (MDR) *Enterobacteriaceae, Enterococci and*/*or Acinetobacter,* in the microbiome of an animal, in particular an avian or mammalian subject, such as a human, comprising administering to said animal, in particular said avian or mammalian subject an effective amount of a bacterium selected from the group consisting of at least one of *E. coli* strains MR010, MK031, MR043, MR045, MR102, MR134, MR158, MK241, MK249, MK267, MK270, MK289, MR107, MR050, MK254, MR174, MR084, MK251, MK062, MR097, MK270, MR138, MK262, andMK179.

It was surprisingly found that isolates of the bacterium *E. coli,* and in particular combinations thereof with *Klebsiella michiganensis* and/or *Klebsiella oxytoca* and thus species related to these strains, constitute an effective probiotic for the prevention and/or treatment of pathogenic and/or antibiotic-resistant *Enterobacteriaceae,* such as *Klebsiella pneumoniae, Enterococci* and/or *Acinetobacter* in the microbiome of an animal, in particular an avian or mammalian subject. Apparently, *E. coli,* particularly in combination with *Klebsiella michiganensis* and/or *Klebsiella oxytoca* and species related to these strains outcompete said *Enterobacteriaceae, Enterococci* and/or *Acinetobacter* and leads to effective decolonization of *Enterobacteriaceae, Enterococci* and/or *Acinetobacter* which is particularly useful in case of multidrug resistant (MDR) *Enterobacteria,* such as *Klebsiella pneumoniae,* and thus causes a prevention and/or treatment of said pathogen(s). The decolonization can both be therapeutic, i.e. after colonization of the gut by the (multi-resistant) pathogen(s), or as a preventive measure before a re-colonization of the gut, as required after antibiotic treatment (see also Examples, below). The prevention and/or treatment of pathogenic and/or antibiotic resistant *Enterobacteriaceae,* such as *Klebsiella pneumoniae, Enterococci* and/or *Acinetobacter* in the microbiome of an animal, in particular an avian or mammalian subject can be preferably embodied as a long-lasting effect in combination with at least one bacterial species selected from the group consisting of Bacteroidetes, Actinobacteria, and Firmicutes spec., and preferably from the families Lachnospiraceae (in particular the genus Schaedlerella, Chodladocola), Acutalibacteraceae (in particular the genus Eubacterium_R), Lactobacillaceae (in particular the genus Ligilactobacillus), Eggerthellaceae (in particular the genus Adlercreutzia), and Rikenelleaceae (in particular genus the Alistipes).

Preferred is therefore the bacterium for use according to the present invention, wherein said prevention and/or treatment is through decolonization of said *Enterobacteriaceae.*

In the context of the present invention, the term "pathogenic" in particular in the context with the *Enterobacteriaceae, Enterococci* and/or *Acinetobacter* as herein, shall generally relate to bacteria that cause undesired infections or inflammations in the gut of a subject.

Preferred examples are hypervirulent strains of *Acinetobacter* or *Klebsiella pneumoniae* strains that can cause IBD.

In the context of the present invention, the term "*Escherichia coli*" or "*E. coli*", in particular in the context with the phrase "bacterium for use" as herein, shall generally relate to the *E. coli* strains MR010, MK031, MR043, MR045, MR102, MR134, MR158, MK241, MK249, MK267, MK270, MK289, MR107, MR050, MK254, MR174, MR084, MK251, MK062, MR097, MK270, MR138, MK262, and MK179, and strains that are related (e.g., because of genetic modifications), but still show the effect of outcompeting the antibiotic resistant *Enterobacteriaceae,* as described herein. "Related" in the context of the present invention further relates to strains that have a genome that is at least 95% identical, preferably at least 99% identical, to the genomes of the strains as indicated in table 1 (see below). The term includes individual strains or isolates or mixtures thereof.

In the context of the present invention, the term "*Klebsiella oxytoca*", in particular in the context with the phrase "bacterium for use" as herein, shall generally relate to the species of the Gram-negative bacterium including species related to *Klebsiella oxytoca,* and includes individual strains or isolates or mixtures thereof. "Related" in the context of the present invention further relates to strains that have a genome that is at least 95% identical, preferably at least 99% identical, to the genomes of the strains as indicated in table 1 (see below). The term shall further include *Klebsiella oxytoca* and species related to *Klebsiella oxytoca* that outcompete the *Enterobacteriaceae* for the metabolic use of beta-glucosidic sugars such as sucrose and/or cellobiose. The term also includes commensal *Klebsiella* strains, *K. michiganensis, K. grimontii,* K. *aerogenes,* and *Klebsiella* that have a carbohydrate utilization pattern substantially identical to *K. oxytoca* strain MK01, and in particular human commensals. *Klebsiella oxytoca* is naturally resistant to ampicillin, amoxicillin, ticarcillin and to antibiotics to which other *Enterobacteriaceae* are also intrinsically resistant (herein referred to as "naturally occurring ampicillin/amoxicillin-resistance phenotype"). Examples in the context of the present invention are the isolates *K. oxytoca* strain MK01 isolated from donor MK1903 (*K. oxytoca* MK01), MR08, *K. oxytoca* strain MK02 isolated from donor MK1901 (*K. oxytoca* MK02), and *K. michiganensis* MR050.

Further preferred is the bacterium for use according to the present invention, wherein the combination is selected from the group *of E. coli* strains MR102, and MK289 with *Klebsiella michiganensis* and/or *Klebsiella oxytoca* MK01, preferably MK289 with *Klebsiella oxytoca* MK01.

Further preferred is the bacterium for use according to the present invention, wherein said *Enterobacteriaceae* is a bacterium that comprises genes (chromosomal and/or extrachromosomal) that encode for at least one multi-drug resistance mechanism, for example OXA-48, NMD-1, NDM-5, OXA-244 or IMP-14 and is selected from at least one of *E. coli* strains ST617 (strain MHH), ST361 (56922), ST940 (56236), ST648 (56589), ST156 (56231), ST167 (55652), ST38 (strain 55838), ST405 (strain 54519), ST1284 (strain 55476), ST1702 (strain 56035), ST131 (strain 21236), ST38 (strain 26260), ST46 (strain 53221), ST617 (strain 54875), ST2197 (strain 44583), ST410 (strain 51853), ST131 (strain PBIO729), ST648 (strain PBIO730), and EPEC (strain O127:H6 strain E2348/69), preferably MHH, 56922, 56231, 21236, 53221, PBIO729, and EPEC (see also https://www.ncbi.nlm.nih.gov/pmc/articles/PMC6113867/ for designations).

Further preferred is the bacterium for use according to the present invention, wherein said species related to *Klebsiella oxytoca* is selected from the group consisting of *K. michiganensis, K. grimontii,* K. *aerogenes,* and *Klebsiella* that are related and/or have a carbohydrate utilization pattern substantially similar to *K. oxytoca* strain MK01.

Further preferred is an *E. coli* strain or a *Klebsiella oxytoca* for use according to the present invention, wherein said strain and/or *Klebsiella oxytoca* for use is a strain or an isolate isolated from a human.

Even further preferred is an *E. coli* strain or a *Klebsiella oxytoca* for use according to the present invention that are genetically modified. The genetic modification can include any suitable modification that does not negatively interfere with the purpose of the strains in the context of the present invention. The genetically modified *E. coli* strain or a *Klebsiella oxytoca* for use according to the present invention may include mutations that improve the safety of the strains, e.g. a mutant lacking functional gene(s) involved in the production of toxins, or lacking genes involved in the pathogenicity of the strain(s) (Darby, Alison et al. "Cytotoxic and pathogenic properties of Klebsiella oxytoca isolated from laboratory animals." PloS one vol. 9,7 e100542. 24 Jul. 2014, doi: 10.1371/journal.pone.0100542).

Resistant *Enterobacteriaceae,* such as *Klebsiella pneumoniae,* in the context of the present invention, shall mean any species or strain (or mixtures thereof) of *Enterobacteriaceae* that has acquired or has acquired an additional resistance phenotype, compared to a sensitive strain (disregarding the naturally occurring resistance phenotypes). Such antibiotic resistance is more available than ever before to organisms such as *Escherichia coli* and *Klebsiella pneumoniae, Enterococci* and/or *Acinetobacter* that are important causes of major sepsis (see, for example, Iredell Jon, Brown Jeremy, Tagg Kaitlin. Antibiotic resistance in Enterobacteriaceae: mechanisms and clinical implications BMJ 2016; 352:h6420). Examples are resistant strains belonging to *Citrobacter, Enterobacter, Hafnia, Klebsiella, Proteus, Providencia, Salmonella,* and *Yersinia.* Additional examples of antibiotic resistant *Enterobacteriaceae* are multi drug resistant *Enterobacteriaceae* (MDR), a concerning problem in clinical environments in both the hospital and the ambulatory setting. Carbapenem-resistant *Enterobacteriaceae* are regarded as an urgent threat by the CDC in the US.

Preferred is therefore the bacterium for use according to the present invention, wherein said *Enterobacteriaceae* is/are multi-resistant *Enterobacteriaceae,* such as *P. mirabilis, E. cloacae,* or *E. coli* and/or *Klebsiella pneumoniae.*

Preferred is an *E. coli* strain or a *Klebsiella oxytoca* for use according to the present invention, wherein said microbiome is located in the gut of said animal, in particular said avian or mammalian subject. Preferred is an *E. coli* strain or a *Klebsiella oxytoca* for use according to the present invention, wherein said microbiome is located in the gut of said animal, in particular an avian or mammalian subject, and wherein said subject is selected from poultry, such as chicken or geese, a mouse, rat, cat, dog, rabbit, goat, sheep, horse, camel, lama, cow, monkey, a farm animal, a sport animal, and a pet, and a human.

The intestinal microbiota plays beneficial roles in many physiological processes of the host. Dysbiosis, a disruption of microbial composition by various stresses, has been implicated in inflammatory bowel disease (IBD), colon cancer, obesity, asthma, and other diseases. In particular, excessive dosing of antibiotics elicits the loss of naturally occurring intestinal microbiota. Such loss increases the numbers of yeasts, such as *Candida albicans,* and bacteria, such as *Proteus, Staphylococcus,* and *Clostridium difficile* (*C. difficile*), that normally exist at low numbers, leading to depression of digestive functions or the occurrence of intestine-related diseases. The idea that probiotics could improve or prevent diarrhea began with the notion that these gut-associated diseases were caused by the collapse of "colonization resistance" due to the absence of normal bacterial flora. Probiotics are known to be very effective for the treatment of antibiotic-associated diarrhea, with *Saccharomyces boulardii* (*S. boulardii*), *E. coli* Nissle 1917, *Lactobacillus,* and *Bifidobacterium* as the main focus of research. Application of FMT and probiotics for eradication of gastrointestinal diseases and enteropathogens exhibits the potential to restore the degraded ecosystem and protection against colonization and proliferation of enteropathogens. Therefore, in another aspect of the present invention, the bacterium is used and/or is for use according to the present invention in reestablishing colonization resistance after antibiotic dysbiosis in the microbiome of an avian or mammalian subject.

Another aspect of the present invention then relates to the bacterium for use according to the present invention, wherein said prevention and/or treatment of said pathogenic and/or antibiotic resistant *Enterobacteriaceae, Enterococci* and/or *Acinetobacter* and/or said reestablishing colonization resistance after treatment or antibiotic dysbiosis is in the context of bacterial infection, bloodstream infection, allogeneic hematopoietic stem cell transplantation, and bacterial invasion into the liver, spleen and/or mesenteric lymph nodes (MLN) in said animal, in particular an avian or mammalian subject. As further described below, luminal and tissue invasion of *K. pneumoniae* MDR1 was assessed in the gastrointestinal organs including small intestine, cecum and colon as well as in the liver, and lymphatic organs including spleen and mesenteric lymph nodes (MLN) on day 6 p.c. A strong reduction in the gastrointestinal organ content as well as in the tissues in both pre-colonized groups compared to the control groups was observed. In addition, *Klebsiella oxytoca* colonized groups did not have any bacteria in the liver, spleen or MLN in contrast to the control animals, suggesting a systemic spread of the pathogen in control mice, elevating the risk for blood stream infections. This experiment demonstrated a broad-spectrum activity of *Klebsiella oxytoca* in all gastrointestinal organs including reduction of pathogenic bacteria in the lumen and tissue and preventing the systemic spread into the liver and lymphatic tissues such as spleen and MLN.

The bacterium for use according to the present invention can be administered to the avian or mammalian subject in any suitable way that allows for colonization, in particular a substantial colonization, with said bacterium, for example as a pharmaceutical composition comprising an effective amount of an *E. coli* strain and/or a *Klebsiella michiganensis* and/or *Klebsiella oxytoca* for use according to the present invention, and a pharmaceutically or therapeutically acceptable excipient or carrier. Compositions are for fecal microbiota transplant (FMT), comprise a suitable medium, such as sterile saline, are used as a suitable solid dosage form, such as a suppository or capsule, in particular stomach-acid resistant, and/or as a probiotic. A combination of the strains (see also above) is explicitly included.

The term "pharmaceutically or therapeutically acceptable excipient or carrier" refers to a solid or liquid filler, diluent or encapsulating substance which does not interfere with the effectiveness or the biological activity of the active ingredients (here, bacteria) and which is not toxic to the host, which may be either humans or animals, to which it is administered. Depending upon the particular route of administration, a variety of pharmaceutically-acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for use according to the present invention and will readily be able to choose suitable pharmaceutically acceptable carriers or excipients, depending, e.g., on the formulation and administration route of the pharmaceutical composition. In a preferred example, the *E. coli* strain and/or the *Klebsiella michiganensis* and/or *Klebsiella oxytoca* for use according to the present invention is provided as a probiotic, i.e. a live microbial food or feed supplement (preparation) which beneficially affects the host animal by improving its intestinal microbial balance.

In addition to the aforementioned bacteria for use according to the invention, the pharmaceutical composition as administered can contain other therapeutically active substances - for example with medicaments already known for the treatment of the aforementioned conditions and/or diseases, in particular suitable nutrients and supplements for bacterial growth as desired, e.g. butyrate, but also suitable antibiotics, whereby in the latter case a favorable additive, amplifying or preferably synergistically effect is noticed.

Also preferred is the *E. coli* strain or the *Klebsiella michiganensis* and/or *Klebsiella oxytoca* for use according to the present invention, wherein said use is in combination with at least one bacterial species selected from the group consisting of Bacteroidetes, Actinobacteria, and Firmicutes spec., and preferably from the families Lachnospiraceae (in particular the genus Schaedlerella, Chodladocola), Acutalibacteraceae (in particular the genus Eubacterium_R) Lactobacillaceae (in particular the genus Ligilactobacillus), Eggerthellaceae (in particular the genus Adlercreutzia), and Rikenelleaceae (in particular genus the Alistipes), and in particular human isolates thereof, and optionally with at least one additional therapeutically active substance as above.

The dosage of the pharmaceutical composition to be administered according to the present invention can be appropriately selected according to the route of administration, the subject to be administered, the target disease and its severity, age, sex weight, individual differences and disease state. Dosage may be repeated several times a day. Preferred is a bacterium for use according to the present invention, wherein said use is in a (single) dosage of between 10¹² and 10⁷ CFU, preferably between 10¹⁰ and 10⁸ CFU of said bacterium, e.g. *an E. coli* strain or a strain of *Klebsiella michiganensis* and/or *Klebsiella oxytoca.* Doses can be applied several times, as needed.

Yet another aspect of the present invention then relates to a method for preventing and/or treating pathogenic and/or antibiotic resistant *Enterobacteriaceae,* such as *E. coli* and/or *Klebsiella pneumoniae,* in particular multi-resistant (MDR) *Enterobacteriaceae, Enterococci* and/or *Acinetobacter* in the microbiome of an animal, in particular an avian or mammalian subject, such as a human, comprising administering to said animal, in particular said avian or mammalian subject an effective amount of a bacterium selected from the group consisting of at least one of *E. coli* strains MR010, MK031, MR043, MR045, MR102, MR134, MR158, MK241, MK249, MK267, MK270, MK289, MR107, MR050, MK254, MR174, MR084, MK251, MK062, MR097, MK270, MR138, MK262, andMK179.

Preferred is the method according to the present invention, wherein said preventing and/or treating is in combination with at least one bacterium selected from the group of *Klebsiella michiganensis* and/or *Klebsiella oxytoca,* in particular from the strain MK01, MR08, MR050, and species related to *Klebsiella oxytoca* MK01.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes but does not require complete recovery or complete prevention of a stress.

As mentioned above, in addition to the bacteria for use according to the invention, the pharmaceutical composition as administered can contain other therapeutically active substances - for example with medicaments already known for the treatment of the aforementioned conditions and/or diseases, in particular suitable nutrients, such as butyrate, and supplements for bacterial growth as desired, but also suitable antibiotics, whereby in the latter case a favorable additive, amplifying or preferably synergistically effect is noticed. Also preferred is method as above, wherein said prevention and/or treatment is in combination with at least one bacterial species selected from the group consisting of Bacteroidetes, Actinobacteria, and Firmicutes spec., and preferably from the families Lachnospiraceae (in particular the genus Schaedlerella, Chodladocola), Acutalibacteraceae (in particular the genus Eubacterium_R), Lactobacillaceae (in particular the genus Ligilactobacillus), Eggerthellaceae (in particular the genus Adlercreutzia), and Rikenelleaceae (in particular genus the Alistipes), and in particular human isolates thereof, and optionally with at least one additional therapeutically active substance as above.

It is to be understood that the present bacteria and/or a pharmaceutical composition comprising the present bacteria is for use to be administered to an animal, such as an avian or mammalian, e.g. a human patient. The term "administering" means administration of a sole therapeutic bacterial agent or in combination with another therapeutic agent. It is thus envisaged that the pharmaceutical composition of the present invention are employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e. directly and/or indirectly, preferably synergistically) with the present compound(s) (for use).

The dosage of the pharmaceutical composition to be administered in the method according to the present invention can be appropriately selected according to the route of administration, the subject to be administered, the target disease and its severity, age, sex weight, individual differences and disease state. Dosage may be repeated several times a day. Preferred is a bacterium for use, such as a strain of *E. coli* and/or *Klebsiella oxytoca* for use according to the present invention, wherein said use is in a (single) dosage of between 10¹² and 10⁷ CFU, preferably between 10¹⁰ and 10⁸ CFU of said strain of *E. coli* and/or *Klebsiella oxytoca.* Doses can be applied several times, as needed.

The prevention and/or treatment of said pathogenic and/or antibiotic resistant *Enterobacteriaceae, Enterococci* and/or *Acinetobacter* and/or said reestablishing colonization resistance after antibiotic dysbiosis can be in the context of bacterial infection, bloodstream infection, allogeneic hematopoietic stem cell transplantation, and bacterial invasion into the liver, spleen and/or mesenteric lymph nodes (MLN) in said animal, in particular an avian or mammalian subject.

According to the present invention, the animal, in particular the avian or mammalian subject to be treated can be preferably selected from poultry, such as chicken or geese, a mouse, rat, cat, dog, rabbit, goat, sheep, horse, camel, lama, cow, monkey, a farm animal, a sport animal, and a pet, and a human.

After establishing different animal models, the inventors isolated promising candidates for a competition with MDR *E. coli* strains. As direct competition for environmental niches in the gut is a fundamental requirement for invading species to successfully establish in the host, which is facilitated after antibiotic perturbation of the resident flora, the inventors aimed at identifying commensal bacterial species as candidates to compete with pathogenic and/or MDR *E. coli* and/or *K. pneumoniae, Enterococci* and/or *Acinetobacter* strains. These strains would also exploit similar nutrient sources and grow under an oxidative environment.

The present invention relates to the following items.

Item 1. A bacterium selected from the group of consisting of at least one of *E. coli* strains MR010, MK031, MR043, MR045, MR102, MR134, MR158, MK241, MK249, MK267, MK270, MK289, MR107, MR050, MK254, MR174, MR084, MK251, MK062, MR097, MK270, MR138, MK262, and MK179 for use in the prevention and/or treatment of antibiotic resistant *Enterobacteriaceae,* such as *E. coli* and/or *Klebsiella pneumoniae,* in particular multi-resistant (MDR) *Enterobacteriaceae, Enterococci* and/or *Acinetobacter* in the microbiome of an animal, in particular an avian or mammalian subject.

Item 2. Bacterium for use according to Item 1, wherein said use is in combination with at least one bacterium selected from the group of *Klebsiella michiganensis and*/*or Klebsiella oxytoca,* in particular from the strain MK01, MR08, MR050, and species related to *Klebsiella oxytoca* MK01, and wherein preferably the combination shows a synergistic effect in the prevention and/or treatment.

Item 3. Bacterium for use according to Item 2, wherein the combination is selected from the group of *E. coli* strains MR102, and MK289 with *Klebsiella oxytoca* MK01, preferably MK289 with *Klebsiella oxytoca* MK01.

Item 4. Bacterium for use according to any one of Items 1 to 3, wherein said antibiotic resistant *Enterobacteriaceae* is selected from at least one of *E. coli* strains MHH, 56922, 56236, 56589, 56231, 55652, 55838, 54519, 55476, 56035, 21236, 26260, 53221, 54875, 44583, 51583, PBIO729, PBIO730, and EPEC, preferably MHH, 56922, 56231, 21236, 53221, PBIO729, and EPEC.

Item 5. Bacterium for use according to any one of Items 1 to 4, wherein said use further comprises the addition of N-acetyl-D-galactosamine.

Item 6. Bacterium for use according to any one of Items 2 to 5, wherein said species related to *Klebsiella oxytoca* is selected from the group consisting of *K. michiganensis, K. grimontii,* K. *aerogenes,* and *Klebsiella* that have a carbohydrate utilization pattern substantially similar to *K. oxytoca* strain MK01, or MR08.

Item 7. Bacterium for use according to any one of Items 1 to 6 in reestablishing colonization resistance after treatment and/or antibiotic dysbiosis in the microbiome of an animal, in particular an avian or mammalian subject.

Item 8. Bacterium for use according to any one of Items 1 to 7, wherein said microbiome is located in the gut of said animal, in particular said avian or mammalian subject.

Item 9. Bacterium for use according to any one of Items 1 to 8, wherein said prevention and/or treatment of said pathogenic and/or antibiotic resistant *Enterobacteriaceae* and/or said reestablishing colonization resistance after treatment and/or antibiotic dysbiosis is in the context of bacterial infection, irritated bowl disease (IBD), bloodstream infection, sepsis, allogeneic hematopoietic stem cell transplantation, and bacterial invasion into the liver, spleen and/or mesenteric lymph nodes (MLN) in said animal, in particular said avian or mammalian subject.

Item 10. Bacterium for use according to any one of Items 1 to 9, wherein said use comprises fecal microbiota transplant (FMT), in a suitable medium, such as sterile saline, or in a suitable solid dosage form, such as a suppository or capsule, in particular stomach-acid resistant, or as a probiotic.

Item 11. Bacterium for use according to any one of Items 1 to 10, wherein said use is in a dosage of between 10¹² and 10⁷ CFU, preferably between 10¹⁰ and 10⁸ CFU of said bacterium.

Item 12. Bacterium for use according to any one of Items 1 to 11, wherein said use is in combination with at least one bacterial species selected from the group consisting of Bacteroidetes, Actinobacteria, and Firmicutes spec., and preferably from the families Lachnospiraceae, such as, for example, Schaedlerella, Chodladocola, Acutalibacteraceae, such as for example Eubacterium_R, Lactobacillaceae, such as, for example, Ligilactobacillus, Eggerthellaceae, such as, for example, Adlercreutzia, and Rikenelleaceae, such as, for example, Alistipes, and in particular human isolates thereof.

Item 13. A method for preventing and/or treating pathogenic and/or antibiotic resistant *Enterobacteriaceae,* such as *E. coli* and/or *Klebsiella pneumoniae,* in particular multi-resistant (MDR) *Enterobacteriaceae, Enterococci* and/or *Acinetobacter* in the microbiome of an animal, in particular an avian or mammalian subject, such as a human, comprising administering to said animal, in particular said avian or mammalian subject an effective amount of a bacterium selected from the group consisting of at least one of *E. coli* strains MR010, MK031, MR043, MR045, MR102, MR134, MR158, MK241, MK249, MK267, MK270, MK289, MR107, MR050, MK254, MR174, MR084, MK251, MK062, MR097, MK270, MR138, MK262, and MK179.

Item 14. The method according to Item 13, wherein said preventing and/or treating is in combination with at least one bacterium selected from the group of *Klebsiella michiganensis* and/or *Klebsiella oxytoca,* in particular from the strain MK01, MR08, MR050 and species related to *Klebsiella oxytoca* MK01 or MR08.

Item 15. The method according to Item 14, wherein the combination is selected from the group of *E. coli* strains MR102, and MK289 with *Klebsiella oxytoca* MK01, preferably MK289 with *Klebsiella oxytoca* MK01.

Item 16. The method according to any one of Items 13 to 15, wherein said antibiotic resistant *Enterobacteriaceae* is selected from at least one of *E. coli* strains MHH, 56922, 56236, 56589, 56231, 55652, 55838, 54519, 55476, 56035, 21236, 26260, 53221, 54875, 44583, 51583, PBIO729, PBIO730, and EPEC, preferably MHH, 56922, 56231, 21236, 53221, PBIO729, and EPEC.

Item 17. The method according to any one of Items 13 to 16, further comprising administering to said animal, in particular said avian or mammalian subject, an effective amount of a bacterium selected from the group consisting of Bacteroidetes, Actinobacteria, and Firmicutes spec., and preferably from the families Lachnospiraceae, such as, for example, Schaedlerella, Chodladocola, Acutalibacteraceae, such as for example Eubacterium_R, Lactobacillaceae, such as, for example, Ligilactobacillus, Eggerthellaceae, such as, for example, Adlercreutzia, and Rikenelleaceae, such as, for example, Alistipes, and in particular human isolates thereof.

The present invention will now be described further in the following examples with reference to the accompanying Figures, nevertheless, without being limited thereto. For the purposes of the present invention, all references as cited herein are incorporated by reference in their entireties.
Figure 1 shows the fold change of the growth of an MDR *E. coli* strain (MHH, ST617) following co-cultivation with several commensal *E.* coli-strains. Individual values of an experiment that was performed in triplicate.
Figure 2 shows in (A) the pre-colonization of several commensal E. coli strains, in (B) the colonization kinetics of an MDR E. coli (strain MHH, ST617) in pre-colonized and control-SPF-mice that were treated with ampicillin, and in (C) the clearance-rates of MDR E. coli MHH in pre-colonized und control animals. Geometric mean of two to three experiments with n=9-17 mice per group.
Figure 3 shows a heatmap of the quantification of the growth of several MDR-E. *coli-*strains following co-cultivation with several commensal *E.* coli-strains or mixtures with *K. oxytoca.* The data are from three independent experiments.
Figure 4 shows in (A) the colonization kinetics des MDR *E*. *coli* (strain 21236, ST131) in pre-colonized und control-SPF-mice that were treated with ampicillin, und in (B) die the colonization kinetics des MDR *E. coli* (strain 55652, ST167) in pre-colonized und control-SPF-mice that were treated with ampicillin. The data are from two to three experiments with n=9-17 mice per group.
Figure 5 shows the colonization kinetics of the strains *E. coli* MR102 and *K. oxytoca* MK01 in a co-colonization in SPF mice that were treated with ampicillin. Data from one experiment with n=5 mice per group.
Figure 6 shows in (A) die pre-colonization of *E. coli* MR102, K. *oxtyoca* MK01 and of both strains in co-colonization, in (B) the colonization kinetics of MDR *E. coli* (strain 55652 ST167) in pre-colonized and control-SPF- mice that were treated with ampicillin, and in (C) the clearance-rates of MDR *E. coli* MHH in pre-colonized and control animals. Geometric mean of two experiments with n=8-10 mice per group.
Figure 7 shows in (A-D) colonization kinetics of different MDR-E strains (A: E. *cloaceae,* B: E. coli ST61 7, C: *K. pneumoniae,* D: P. mirabilis) and in (E) of S. Typhimurium. Geometric mean of two experiments with n=9-10 mice per group.
Figure 8 shows the area under the curve (AUC) of growth curves of different strains of E. coli in minimal medium, supplemented with different sugars. The results are depicted as a heatmap of the AUC after 72 hours of incubation.

### EXAMPLES

### Material and Methods

### Human cohorts

Human sample and data collections have been performed in agreement with the guidelines of the Helmholtz Center for Infection Research, Braunschweig, Germany, the Ethics Committee Lower Saxony (permit No. 8629_BO_K_2019; No. 8750_BO_K_2019) and the European Data Protection Laws (Europäische Datenschutz-Grundverordnung DSGVO). All human donors have signed a letter of informed consent by the World Medical Association Declaration of Helsinki (Version 2013).

### Mice

All animals which were used for in vivo experiments were gender and age-matched, female and male mice with an age of 8-16 weeks and a weight of 16-30 g at the beginning of the experiment. Mice were kept under a 12 h light cycle (lights on from 7 am to 7 pm). C57BL/6N mice were bred at the animal facility of the Helmholtz Centre of Infection Research. SPF mice were bred under specific pathogen-free (SPF) conditions (Stehr et al. 2009) and germ-free C57BL/N6Tac mice were bred in isolators (Getinge). Mice were killed by euthanization with CO₂ and cervical dislocation.

### Bacterial strains

Multidrug-resistant (OXA-48 carbapenemase) *E. coli MDR1* (2365332, ST617), obtained from the Hannover medical school, and isolated from a rectal swab of a patient, was used for most in vivo colonization experiments and in vitro assays. Multidrug-resistant *E. coli* NRZ-21236 and NRZ-55652, obtained from the National Reference Center for Multidrug-resistant Gram-negative Bacteria (Bochum) was used for two in vivo colonization experiments. Further commensal *E. coli* isolates used for in vivo and in vitro competition experiments were isolated from the human cohort studies at the HZI (MikroResist=MR, MikroKids=MK, LöwenKids=LK, RheumaVor=RV).

### Strain isolation

Fresh stool samples were homogenized by bead-beating in a Mini-Beadbeater (BioSpec) with 1 mm zirconia/ silica beads in 1 ml PBS for 50 s. Stored fecal samples were directly taken from glycerol stock. All samples were serially diluted and plated on CHROMagar Orientation plates and MacConkey agar plates. Colonies were isolated and identified by 16S-PCR and whole genome sequencing.

### Quantification of E. coli colony-forming units

To determine the CFU of bacteria from culture or feces samples under different conditions, samples were spread on plates. Fresh fecal samples were collected and weighted for quantification of CFUs from fecal samples. Afterward, samples were prepared by adding 1 mm Zirconia beads and 1 ml PBS. Samples were homogenized by bead-beating for 50 s (Mini-Beadbeater, BioSpec). To determine CFUs, 25µl of serial dilutions of samples were plated on LB plates with respective antibiotics or MacConkey agar supplemented with Maltose. Plates were cultivated overnight at 37 °C under aerobic conditions before counting. Colony counts were normalized to plated volume and/ or weight of feces.

### Ex vivo competition in germ-free cecum content

Germ-free mice were sacrificed, and cecal contents were isolated and diluted 1: 1 in PBS. Bacteria were grown in 5 ml LB for 16 h at 37 °C under aerobic conditions before adjusting the OD₆₀₀. MDR *E. coli* strains were adjusted to OD₆₀₀ of 1, and the commensal strains were adjusted to OD₆₀₀ of 0.2. The assay was performed in 96-well plates with 250 µl diluted cecum content with co-cultures of 20 µl of commensal and 10 µl of MDR *E. coli* strain. Single culturing of the MDR *E. coli* strain served as a control.

### In vitro competition for carbohydrates

To investigate direct competition or specific carbohydrates, bacteria were co-cultivated in MM9 media supplemented with 5 g/L of the respective carbon source. To do so, bacterial strains were cultivated on R2A agar plates (Difco) overnight, and bacteria were adjusted to an OD600 of 0.2 in PBS. 99 µl of MM9 media with the respective carbon source was filled into a 96-well plate, and 1 µl of each strain was added. Co-cultures were cultivated under aerobic conditions at 37 °C for 24 h. After incubation, CFUs were determined as previously described.

### Phenotypic microarrays

For carbohydrate utilization screenings, the phenotypic microarrays PM1 and PM2a from Biolog^{™} were used. Bacterial strains were cultivated on R2A agar plates (Difco) overnight. Colonies were scraped and adjusted to an OD₆₀₀ of 0.2 in PBS. Each well of the PM1 and PM2a plates was filled with 99 µl of MM9 without carbon sources and inoculated with 1 µl of bacterial suspension. For competition experiments, each well was inoculated with 1 µl of bacterial suspension of each strain. The plates were incubated aerobically in a microplate spectrophotometer with continuous shaking, OD₆₀₀ was measured hourly for 24 h. Mixtures were plated on selective agar plates as previously described.

### Growth analysis

Bacterial strains were cultivated on R2A agar plates (Difco) overnight. Colonies were scraped and adjusted to OD₆₀₀ of 0.2 in PBS. Subsequently, a flat-bottomed 96-well plate (Coming^{®}) was filled with 99 µL of MM9 medium supplemented with the respective carbon source and inoculated with 1 µL of the bacterial suspension. Sterile media served as a negative control. Plates were incubated in a microplate spectrophotometer with continuous shaking (BioTek LogPhase 600 Microbiology Reader). OD600 was measured hourly over a time course of 72 hours.

### In vivo E. coli colonization

SPF mice were treated with ampicillin (0.5 g/l) 4 d before starting the experiment. OMM¹² and OMM¹⁹ mice did not receive ampicillin. On the day of colonization, respective bacteria were cultivated in 25 ml LB media (1:25) at 37 °C for 3-4 h. Afterwards, the culture was centrifuged at 500 g for 15 min. The pellet was suspended in 10 ml of PBS and the required amounts were calculated. Mice were inoculated by oral gavage of 10⁸ CFUs of *E. coli* diluted in 200 µl PBS. The body weight of the mice was monitored, and feces were collected at different time points (1, 3, 6, 9, 14, 21, 28, and 42 days after gavage) to measure fecal *E. coli* burden by plating and 16S rRNA sequencing.

### In vivo decolonization experiments

SPF mice were treated with ampicillin (0.5 g/l) 4 d before starting the experiment. OMM¹² and OMM¹⁹ mice did not receive ampicillin. On the day of colonization, bacterial cultures were prepared as described previously. Mice were precolonized by oral gavage with 10⁸ CFUs/ 200 µl in PBS. 4 days after precolonization fecal CFUs of respective *E. coli* strains were checked by plating on selective agar plates. After successful precolonization (CFU/g of at least 10⁹ CFUs), the competing *E. colil* MDR-E strain was administered by oral gavage (10⁸ CFUs in 200 µl PBS). The body weight of the mice was monitored, and feces were collected at different time points (1, 3, 6, 9, 14, 21, 28, and 42 days after gavage) to measure fecal *E. coli* burden by plating and 16S rRNA sequencing.

### DNA isolation

The microbial community 16S rRNA gene DNA was extracted using precipitation with phenol-chloroform. 0.1 mm Zirconia beads were added to the feces sample until the bottom part of the tube was covered. Afterwards, 500 µl of 2× Buffer A and 200 µl of 20 % SDS were added to each sample. Then 500 µl of the bottom phase of Phenol: Chloroform: IAA was added. This mixture was shaken by a bead-beater for 2 min, then cooled down at 4 °C for 2 min, and then again shaken for 2 min. For phase separation, samples were centrifuged at 8000 rpm for 5 min at 4 °C. The aqueous phase was transferred to a fresh 1.5 ml tube and 600 µl of Phenol: Chloroform: IAA was added and mixed by inverting samples several times. Then, samples were centrifuged at 12700 rpm for 5 min at 4 °C for the second phase separation. Again, the aqueous phase was transferred to a fresh 1.5 ml tube. 600 µl of 100 % Isopropanol (stored at -20 °C) and 60 µl (1/10 volume) of 3 M NaOAc (pH = 5.5) were added. The mixture was vortexed and then stored at -20 °C for at least 1 h or overnight. After incubation at -20 °C, samples were centrifuged at 12700 rpm for 20 min at 4 °C. Then, all liquid was sucked off. The remaining pellet was washed with 1 ml of 70 % EtOH (RT) and centrifuged at 12700 rpm for 5 min at 4 °C. Afterwards all liquid was sucked off carefully and the pellet was dried in a speedvac machine for 15 min using no heat. The pellet was resuspended in 100 µl TE-buffer and incubated at 50 °C for 30 min on a shaker at 1000 rpm. When the pellet was completely dissolved, 1 µl of 10 µg/ ml RNAse was added. Crude DNA was column purified (BioBasic Inc.) to remove PCR inhibitors.

### 16S rRNA gene amplification and sequencing

16S rRNA gene amplification of the V4 region (F515/R806) was performed according to an established protocol previously described ⁶⁰. Briefly, DNA was normalized to 25 ng/µl and used for sequencing PCR with unique 12-base Golary barcodes incorporated via specific primers (obtained from Sigma). PCR was performed using Q5 polymerase (NewEnglandBiolabs) in triplicates for each sample, using PCR conditions of initial denaturation for 30 s at 98°C, followed by 25 cycles (10 s at 98°C, 20 s at 55°C, and 20 s at 72°C). After pooling and normalization to 10 nM, PCR amplicons were sequenced on an Illumina MiSeq platform via 300 bp paired-end sequencing (PE300). Using the Usearch8.1 software package (http://www.drive5.com/usearch/) the resulting reads were assembled, filtered, and clustered. Sequences were filtered for low-quality reads and binned based on sample-specific barcodes using QIIME v1.8.0. Merging was performed using -fastq_mergepairs - with fastq_maxdiffs 30. Quality filtering was conducted with fastq_filter (-fastq_maxee 1), using a minimum read length of 250 bp and a minimum number of reads per sample = 1000. Reads were clustered into 97% ID OTUs by open-reference OTU picking and representative sequences were determined by use of the UPARSE algorithm (Edgar 2010). Abundance filtering (OTUs cluster >0.5%) and taxonomic classification were performed using the RDP Classifier executed at 80% bootstrap confidence cut-off. Sequences without matching reference datasets, were assembled as de novo using UCLUST. Phylogenetic relationships between OTUs were determined using FastTree to the PyNAST alignment (Price, Dehal, and Arkin 2010). The resulting OTU absolute abundance table and mapping file were used for statistical analyses and data visualization in the R statistical programming environment package phyloseq.

### Whole genome sequencing

To assess the taxonomy of all *E. coli* isolates, bacteria were sent for whole genome sequencing. First, genomic DNA was extracted using the ZymoBIOMICS 96 MagBead DNA Kit according to the manufacturer's instructions. Afterwards, libraries of each isolate were prepared using the NEBNext^{®} Ultra^{™} DNA Library Prep Kit for Illumina^{®}. DNA was fragmented, ligated to adaptors, enriched, and assessed on a Bioanalyzer. Afterwards, libraries of each isolate were prepared using the Illumina DNA PCR-Free Prep and quantified with KAPA Library Quantification Kit Illumina Platforms. Samples were pooled and sent for whole genome sequencing performed by the group of Genome analysis at Helmholtz-HZI Center for Infection Research using NovaSeq 6000 S4 Reagent Kit v1.5 (300 cycles) and targeting depth of 1 million reads per sample. The resulting *E. coli* genomes have been deposited in GenBank (see Data availability).

### Identification of virulence-associated genes (VAGs), AMR, and colibactin genome islands

The inventors identified genes related to virulence and antimicrobial resistance by running the abritamr tool (v1.0.14) on the nucleotide sequences of the isolates. In order to compare the commensal isolates from this invention to pathogenic ones, the inventors performed the same analysis on a collection of 912 clinical *E. coli* isolates from patients with bloodstream infections. The inventors further identified isolates carrying the colibactin genome island (pks+) following a similar approach developed previously. The inventors aligned the proteomes of all isolates against the protein sequences of the clb genes encoded in *E. coli* strain IHE3034 (GenBank accession AM229678.1), using Blast+ v2.12.0 with an e-value threshold of 1·10-4, a query and subject coverage > 80% and protein sequence identity > 70%. The inventors flagged an isolate to be pks+ if the inventors found a homolog of 16 of the 18 clb genes and if the average nucleotide distance between genes encoded in the same contig was below 5kbp.

### Association analysis

To facilitate the annotation of association hits the inventors included 8 *E. coli* closed reference genomes from RefSeq, downloaded using the ncbi-genome-download package v0.3.3. The inventors included the following genomes: 536 (GCF_000013305.1), CFT073 (GCF_000007445.1), ED1a (GCF_000026305.1), IAI1 (GCF_000026265.1), IAI39 (GCF_000026345.1), MG1655 (GCF_000005845.2), UMN026 (GCF_000026325.1), and UTI89 (GCF_000013265.1). For each genome the inventors assigned their sequence type (ST) and Escherichia phylogroup using mist v2.16 and ClermontTyping (commit 740c59c), respectively. The inventors generated a de Bruijn graph from all input genomes and extracted unitigs and their presence/absence patterns across all samples using unitig-counter v1.1.0.^{71,72} Each unitig with allele frequency < 1 encodes for a genetic variant, which can be a large one such as a gene transferred horizontally, as well as short variants such as single nucleotide polymorphisms (SNPs). In order to run associations between variants and the protective phenotype, a kinship matrix is required; this can be derived from a core genome alignment, which the inventors generated by first computing the pangeome with panaroo v1.3.0, using the "--clean-mode strict" argument, followed by a concatenation of individual alignments for each nucleotide coding sequence belonging to core genes (i.e. with frequency >= 95%). The inventors then used snp-sites v2.5.1 and bcftools v1.13 to convert the full core genome alignment to a VCF file containing the variant sites with minimum allele frequency (MAF) > 1%, and used this file to derive the kinship matrix using a python script.

The inventors estimated narrow-sense heritability for the protective phenotype, using two different covariance matrices; one built from the STs of each strain ("lineage") and another using the kinship matrix described above. The inventors used Limix v3.04, assuming normal errors for the point estimate and the inventors computed the 95% confidence intervals using the ALBI package (commit 90d819e).

The inventors then tested the association between each unitig with MAF > 1% and the protective phenotype using a linear-mixed model as implemented in pyseer v1.3.6. The inventors determined an appropriate significance threshold by counting the number of unique unitigs presence/absence patterns tested, which reduces the risk of excessively deflating association p-values. The inventors mapped the unitigs passing the significance threshold back to all input genomes using bwa v0.7.17 and bedtools v2.31.1, using the output of panaroo to assign each unitig to a gene cluster. The unitigs were further filtered to reduce the number of spurious associations: unitigs were excluded if they were shorter than 30bp, if they were mapped to multiple locations in each individual genome, if they mapped to less than 9 samples (~2% of the sample size) and if they were mapped to more than 10 different genes across all samples. The inventors further computed the odds ratio for each gene cluster using the average frequency and effect size as input for a previously developed method for binary phenotypes.

The inventors further annotated the gene families with mapped unitigs by taking a representative protein sequence from the genomes encoding each gene family, giving priority to the 8 closed reference genomes, and using them as an input for eggnog-mapper v2.1.3. The derived annotations include COG categories, GO terms and mapping to KEGG entries; the inventors tested an enrichment for each of these annotation systems using the associated gene families as foreground and the annotation for *E. coli* IAI39 as the background, running a Fisher's exact test for each annotation item, and the inventors used an FDR-corrected p-value threshold of 0.05 to indicate annotation items enriched in the associated gene families.

The inventors generated a phylogenetic tree of all samples using the concatenated core genome alignment as an input for FastTree v2.1.11, using the GTR+CAT model. The tree and associated metadata was visualized using the microreact web interface.

**Table 1: Overview over strains as used in the context of the present invention. Several of the strains were deposited at the Leibniz Institute, DSMZ-German Collection of Microorganisms and Cell Cultures GmbH, Inhoffenstraße 7B, 38124 Braunschweig, Germany on May 24, 2024 under the numbers as indicated. The Biosample numbers refer to the Bioproject PRJEB76066 (for E. coli), and PRJEB42167 for MK01, respectively (website https://www.ncbi.nlm.nih.gov/bioproject).**

| | | | | | | | | |
|---|---|---|---|---|---|---|---|---|
| Indicated is furthermore the competitive "strength" of the strains, as "fold change to control", i.e. compared to a single culture of a respective MDR strain. | | | | | | | | |
| The genomes of the Klebsiella strains are also disclosed in Osbelt, L., Almási, É.d.H., Wende, M. et al. Klebsiella oxytoca inhibits Salmonella infection through multiple microbiota-context-dependent mechanisms. Nat Microbiol (2024). | | | | | | | | |
| https://doi.org/10.1038/s41564-024-01710-0. | | | | | | | | |
| The phylogroup refers to the groups as described in Jolley, K. A. & Maiden, M. C. J. (in: BIGSdb: Scalable analysis of bacterial genome variation at the population level. BMC Bioinformatics 11, (2010)), and Beghain, J., et al. (in: Typing: An easy-to-use and accurate in silico method for Escherichia genus strain phylotyping. Microb. Genomics 4, 1-8 (2018)). | | | | | | | | |
| The determination of virulence genes and antimicrobial resistance (AMR) genes was made according to Sherry, N. L. et al. (in: An ISO-certified genomics workflow for identification and surveillance of antimicrobial resistance. Nat. Commun. 14, (2023)), and Denamur, E. et al. (in: Genome wide association study of Escherichia coli bloodstream infection isolates identifies genetic determinants for the portal of entry but not fatal outcome. PLoS Genet. 18, 1-20 (2022)). | | | | | | | | |

| DSM Number | Species | Isolate | Biosample Number | fold change to control | ex vivo phenotype | Phylo-group | Number virulence genes | Number AMR genes |
|---|---|---|---|---|---|---|---|---|
| DSM 35034 | E. coli | MR010 | ERS20121687 | 0.057503356 | non-competitive | D | 4 | 10 |
| DSM 35035 | E. coli | MK031 | ERS20121681 | 0.077852349 | non-competitive | A | 0 | 3 |
| DSM 35036 | E. coli | MR043 | ERS20121688 | 0.009395973 | competitive | B1 | 16 | 10 |
| DSM 35037 | E. coli | MR045 | ERS20121689 | 0.062120805 | non-competitive | A | 1 | 4 |
| DSM 35038 | E. coli | MR102 | ERS20121690 | 0.008577181 | competitive | D | 22 | 15 |
| DSM 35039 | E. coli | MR134 | ERS20121691 | 0.004308725 | competitive | B1 | 3 | 5 |
| DSM 35040 | E. coli | MR158 | ERS20121692 | 0.019677852 | competitive | B1 | 18 | 11 |
| DSM 35041 | E. coli | MK241 | ERS20121682 | 0.044008715 | competitive | B1 | 3 | 5 |
| DSM 35042 | E. coli | MK249 | ERS20121683 | 0.026143791 | competitive | D | 5 | 6 |
| DSM 35043 | E. coli | MK267 | ERS20121684 | 0.090631808 | non-competitive | B1 | 6 | 4 |
| DSM 35044 | E. coli | MK270 | ERS20121685 | 0.081263617 | non-competitive | B1 | 4 | 5 |
| DSM 35045 | E. coli | MK289 | ERS20121686 | 0.053281853 | non-competitive | B2 | 17 | 5 |
| - | E. coli | MR107 | ERS20167202 | 0.052702703 | non-competitive | B2 | 23 | 14 |
| - | E. coli | MR050 | ERS20167195 | 0.059194631 | non-competitive | B1 | 18 | 11 |
| - | E. coli | MK254 | ERS20167172 | 0.062091503 | non-competitive | B2 | 15 | 10 |
| - | E. coli | MR174 | ERS20167212 | 0.062816735 | non-competitive | D | 18 | 13 |
| - | E. coli | MR084 | ERS20167197 | 0.066442953 | non-competitive | B2 | 23 | 6 |
| - | E. coli | MK251 | ERS20167171 | 0.067320261 | non-competitive | B2 | 18 | 7 |
| - | E. coli | MK062 | ERS20167141 | 0.075302013 | non-competitive | B2 | 21 | 7 |
| - | E. coli | MR097 | ERS20167200 | 0.08 | non-competitive | D | 14 | 9 |
| - | E. coli | MR094 | ERS20167199 | 0.100134228 | non-competitive | B2 | 9 | 7 |
| - | E. coli | MR138 | ERS20167205 | 0.087516779 | non-competitive | A | 2 | 9 |
| - | E. coli | MK262 | ERS20167174 | 0.094488017 | non-competitive | B2 | 21 | 8 |
| - | E. coli | MK179 | ERS20167144 | 0.098408957 | non-competitive | E | 8 | 7 |
| - | K. oxytoca | MK01 | ERS6679003 | NA | competitive | NA | NA | 1 |
| - | K. oxytoca | MR08 | ERS 18097640 | NA | competitive | NA | NA | 1 |
| - | K. | MR050 | ERS20227797 | NA | competitive | NA | NA | 1 |
| | michiganensis | | | | | | | |

### Commensal E. coli strains show different competitive effects against MDR E. coli in an ex vivo screening assay

Since the species *E. coli* is characterized by a large genomic diversity, as many strains as possible were screened. To enable a screening of 430 strains, the inventors found that the *ex vivo* setup is the most feasible. Thus, to characterize the commensal *E. coli* strains from the generated strain collection regarding their ability to compete with an MDR *E. coli* strain, the inventors utilized an *ex vivo* screening assay (1). Co-cultivation in cecum content of germ-free (GF) animals under microaerophilic conditions enables to mimic the gut environment and nutritional landscape of an in vivo setting without the influence of a resident microbiota. The inventors co-cultivated the human-derived commensal *E. coli* strains from the strain collection together with one clinical isolate of a multi-drug resistant (MDR) *E. coli* strain (strain MHH, patient isolate, ST617, origin: Hanover) in a 10:1 ratio (commensal: MDR) in the isolated cecal contents diluted in PBS, for 24 h at 37°C. The inventors used a single culture of the *E. coli* MHH in GF cecum content as a growth control and quantified the CFU/ml of the *E. coli* MHH in single and co-cultures by plating on selective agar plates. To present the results of this assay, the fold change of the co-cultures to the growth control was calculated. Here, a fold change of 1 means no influence on the growth of the *E. coli* MHH, a fold change of <1 means a reduction of growth, and a fold change of >1 means increased growth compared to the growth control.

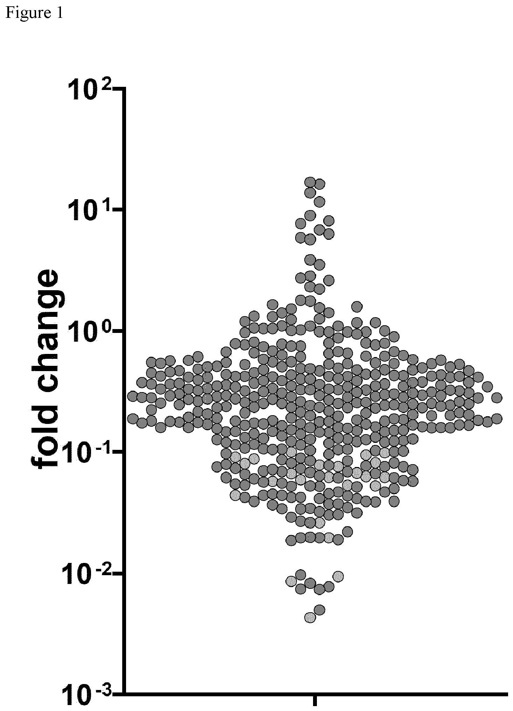

The inventors could observe a broad range of fold changes after co-cultivation with different commensal isolates. The fold change ranged from 16.8 (least competitive) to 0.004 (most competitive). The majority of strains showed a fold change of 1 to 0.1. (Figure 1). Comparing the four cohorts to each other, a similar pattern could be observed for each cohort, indicating that competitive or non-competitive effects are generally distributed and not linked to a specific cohort or age group.

### Specific commensal E. coli strains enable the decolonization of an MDR E. coli strain in a preventive manner in vivo

To elucidate the potential protective effects shown in the *ex vivo* assay *in vivo,* the inventors selected a panel of commensal *E. coli* strains exhibiting different competitive effects. An SPF mouse model with ampicillin treatment was chosen to validate the results in vivo. This model was used to investigate the competitive properties of Enterobacteriaceae before (1). Due to ampicillin treatment, strains with intrinsic ampicillin resistance were selected. The inventors selected the strains *E. coli* MR102, MR158, and RV228 as they belonged to the strains with a fold change below 0.02, thus showing a strong competition, as competitive strains and *E. coli* strains MR103, LK91, MK192, and LK192 as non-competitive strains (Fold-change >0.1) (Figure 1, strains marked in light gray). Mice were treated with 0.5 g/L ampicillin in their drinking water for four days before pre-colonization with 10⁸ CFUs of the respective commensal strain by oral gavage. Another group was treated with PBS as a control. After four days, pre-colonization levels were monitored by plating of fecal samples on selective agar plates and all mice were challenged by oral gavage with 10⁸ CFUs of the MDR *E. coli* MHH. Afterward, fecal colonization levels of the MDR *E. coli* MHH were monitored on days 1, 3, 6, 9, 14, 21, 28, and 42 by plating of fecal samples on selective agar plates. Ampicillin treatment was stopped on day three after administration of the MDR *E. coli* MHH, and mice were switched back to regular drinking water.

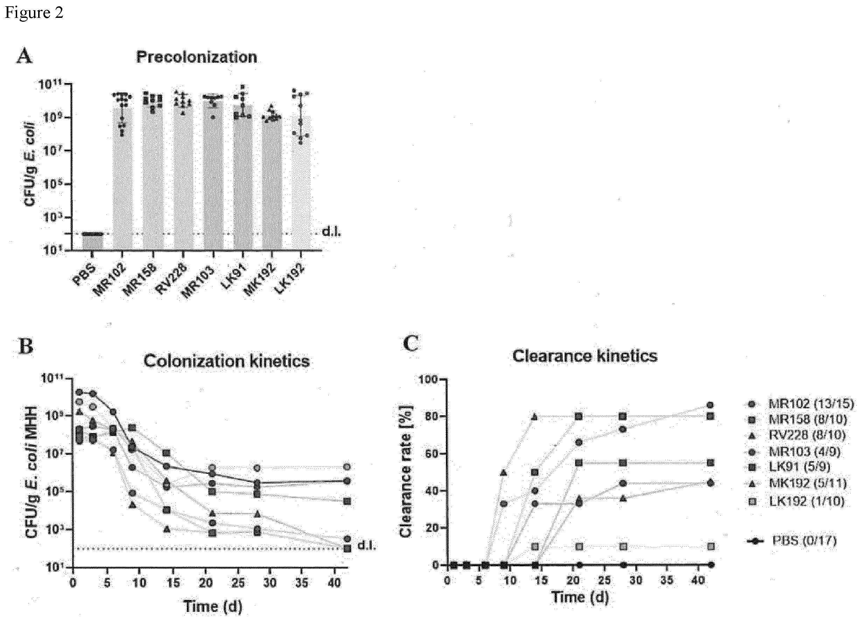

A comparison of the pre-colonization levels of all strains shows that all strains can colonize mice to mean values of 10⁹ to 10¹⁰ CFU/g (Figure 2A). Monitoring the fecal colonization kinetics, the inventors observed that pre-colonization with different commensal *E. coli* strains results in different colonization kinetics of the MDR *E. coli* MHH strain, compared to the control group (Figure 2B). It was observed that groups pre-colonized with *E. coli* strains MR102, MR158, RV228, MR193, LK91, and MK192 showed a 100-fold reduction of CFUs of MDR *E*. *coli* MHH by day three and a 10-fold reduction in the group pre-colonized with *E. coli* LK192, compared to the PBS treated group. On day nine, clearance in the groups pre-colonized with competitive isolates could be observed. Five out of 15 mice pre-colonized with MR102 and five out of ten mice pre-colonized with RV228 could clear out the MDR *E. coli* MHH from feces. This effect is increased at day 28 with clearance rates of 70-80 % in groups pre-colonized with competitive isolates. In the groups pre-colonized with *E. coli* MR103, LK91, and MK192 clearance rates of 40- 60 % were observed, whereas mice pre-colonized with *E. coli* LK192 showed a clearance rate of 10 %, and only 1/10 mice could clear the MDRE. *coli* MHH below the detection limit (Figure 2C).

These experiments support the inventor's hypothesis that specific commensal *E. coli* isolates may harbor competitive effects, which are not exhibited by all strains. Furthermore, it was demonstrated that the conducted *ex vivo* assay is a valid tool for pre-screening of potential probiotic strains. The inventors selected the *E. coli* MR102 as a representative strain with probiotic potential and used it for further experiments.

### Analysis of carbohydrate utilization patterns of MDR and commensal E. coli strains

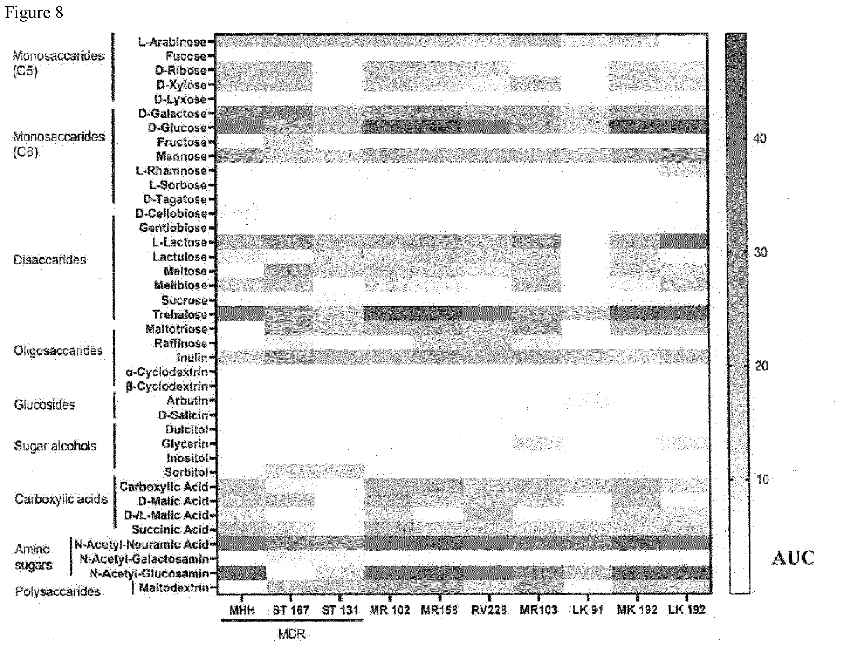

To elucidate the ability of different *E. coli* strains to utilize various carbon sources, the inventors performed growth analysis of representative *E. coli* strains classified as MDR (MHH, 21236, 55652) since they were included in the following in vivo competition experiments and commensal strains, which were previously screened for their competitive phenotypes *in vivo* (MR102, MR158, RV228, MR103, LK91, MK192, LK192). Growth curves were performed in basal minimal media (MM9) over 72 h in a microplate reader under aerobic conditions. Bacteria were grown overnight on R2A agar plates to ensure the loss of storage of nutrients. Afterwards, cultures were diluted and inoculated in MM9 supplemented with 5 g/L of the respective carbon source. The area under the curve (AUC) was calculated and different carbohydrate utilization capabilities by different *E. coli* strains were observed. The strains investigated here displayed different carbohydrate utilization patterns (Figure 8). Among the MDR strains, MDR *E. coli* MHH (ST617) was able to grow in 20 out of the 39 carbon sources tested, while MDR *E. coli* 55652 (ST167) exhibited growth in 24 carbon sources and MDR *E. coli* 21236 (ST131) showed growth in 17 carbon sources. Competitive strains *E. coli* MR102, *E. coli* MR158, and *E. coli* RV228 utilized 22, 21, and 22 carbon sources, respectively. Intermediate and non-competitive strains displayed highly variable utilization patterns. *E. coli* MR103 was able to utilize up to 24 carbon sources, while *E. coli* LK91 could only utilize 13 carbon sources in total and *E. coli* MK192 was able to utilize 21 out of the 39 tested carbon sources and *E. coli* LK192 was able to utilize 20 carbon sources. Visualizing the number of carbon sources, which resulted in strong growth (AUC >20) for each strain, *E. coli* MR102 could utilize the most carbon sources with AUC >20 (n=15).

To focus on the utilization patterns of *E. coli* MHH, MR102, and LK192, the inventors observed a significant degree of overlap (15/39 tested carbon sources). Furthermore, *E. coli* MR102 shows an overlap in utilized carbon sources with MDR *E. coli* MHH (D-malic acid, lactulose) and *E. coli* LK192 (maltotriose, maltodextrin, maltose). Moreover, distinct utilization patterns become evident, as certain sugars were exclusively metabolized by the single strains. D-cellobiose, D-salicin, and D-fructose could solely be utilized by MDR *E. coli* MHH, N-acetyl-D-galactosamin by *E. coli* MR102, and L-rhamnose by *E. coli* LK192.

### In vivo supplementation with cellobiose diminishes the protective effect

To investigate whether carbohydrate competition is involved in the protective effect of *E. coli* MR102 against MDR *E. coli* MHH *in vivo,* mice were supplemented with cellobiose, a carbon source that can only be utilized by MDR *E. coli* MHH, but not *E. coli* MR102.

Sugar supplementation was started with the start of ampicillin treatment at day -7 and continued until day 14. The fecal burden of MDR *E. coli* MHH in cellobiose-treated and *E. coli* MR102 pre-colonized mice was compared to PBS and *E. coli* MR102-treated mice without sugar supplementation. CFU/g of MDR *E. coli* MHH were significantly higher in cellobiose-treated groups compared to *E. coli* MR102 control on day 14, reaching a 10,000-fold difference on day 42. On top of that, clearance of *E. coli* MR102 was significantly delayed and decreased in cellobiose-treated mice. Mice pre-colonized with *E. coli* MR102 and supplemented with cellobiose reached a final clearance rate of 50 %, compared to 80 % in the non-cellobiose treated group.

To investigate whether cellobiose supplementation affects *E. coli* MR102 colonization and thereby interferes with the protective effect, the inventors monitored the fecal colonization levels of *E. coli* MR102 with or without cellobiose treatment at different time points of the experiments. No differences could be observed between both groups for the pre-colonization levels and the day six levels. Notably, at day 28 colonization of *E. coli* MR102 was rather slightly increased in cellobiose-treated mice. This leads to the hypothesis that the diminished protective effect is not caused by lower colonization levels of protective *E. coli* MR102.

### Evaluation of the strain specificity of commensal isolates and the potential to broaden the target spectrum by a combination of different strains

### The competitive effect of different commensals is strain-specific

Since strains belonging to the species *E. coli* are characterized by a large genomic variability and diversity, including between MDR *E. coli* strains, the inventors wanted to assess whether the protective effect is strain-specific. To investigate the competitive effects of commensal isolates against a variety of strains, the inventors conducted an ex vivo assay, screening different commensal isolates against a panel of 18 MDR *E. coli* strains and one EPEC strain. 16 of the MDR strains represent different STs associated with infections and gut colonization in a hospital setting and were isolated from rectal swabs or human fecal samples. Two additional strains isolated from a dog and a crow, respectively, and one EPEC strain were included. The inventors co-cultivated these strains together with either one of four commensal *E. coli* strains from the strain collection (MR102, MR103, MR134, and MK289) or *E. coli* Nissle. Furthermore, the inventors included *K. oxytoca* MK01, which was previously described to have the ability to promote gut decolonization of MDR *K. pneumoniae* (1), as well as mixtures of the commensal *E. coli* strains and *K. oxytoca* MK01.

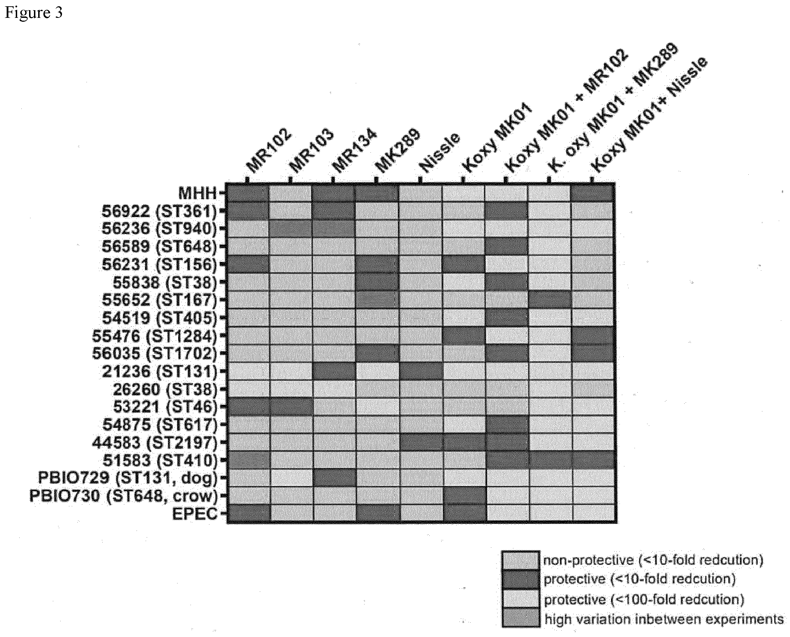

The respective commensal strains were classified by the ability to reduce the CFUs <10-fold (non-competitive), <10-fold (intermediate), or <100-fold (competitive) (Figure 3). The inventors found that the commensal strains show different competitive effects. *K. oxytoca* MK01 could reduce the CFUs of most of the strains (12/19), followed by the *E. coli* strains MR102 and MK289 (7/19), then MR103 and MR134 (5/19). The probiotic *E. coli* Nissle could only reduce the CFUs of two of the MDR strains. Interestingly, the inventors could identify one MDR strain (21236, ST131) that was reduced by all of the strains and one MDR strain (55652, ST167), which could not be reduced by any of the tested isolates. Moreover, the spectrum of competitive effects of each commensal could be broadened by combining commensal *E. coli* strains with *K. oxytoca* MK01. A mixture of *K. oxytoca* MK01 and *E. coli* MK289 could reduce the CFUs of all 19 strains and the combination of *K. oxytoca* MK01 and *E. coli* MR102 could reduce the CFUs of 84% (16/19) of the tested strains.

Surprisingly, the combination of *K. oxytoca* MK01 and *E. coli* Nissle showed the least competitive effects and could reduce the CFUs of only 13 of the 19 tested strains.

Together, these experiments showed that combinations of specific commensal strains could broaden the protective effect.

### E. coli MR102 is able to compete with MDR E. coli 55652 (ST167) and 21236 (ST131)

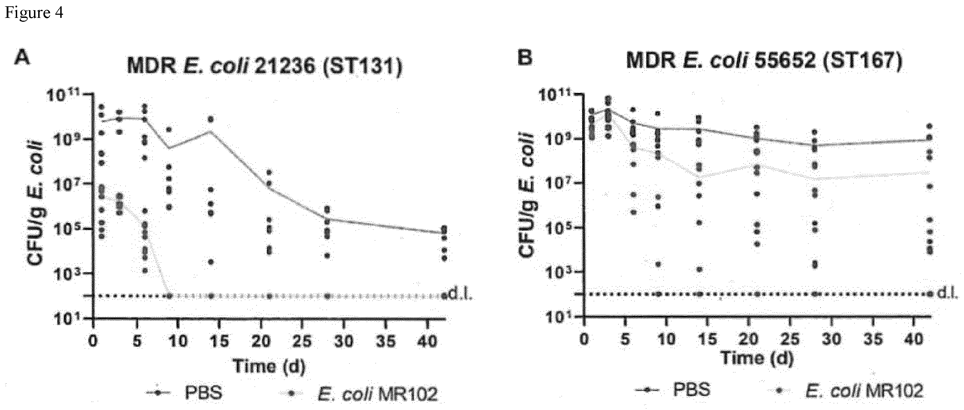

To validate the results of the ex vivo assay, two MDR *E. coli* strains were selected for in vivo experiments. The strain *E. coli* 21236 (ST131) was selected since ST131 is the most prevalent and urgent sequence type of MDR E. coli strains regarding colonization and infections in hospital settings5,6. In addition, the strain *E. coli* 55652 (ST167) was selected, because it could not be reduced by any of the commensal *E. coli* strains or *K. oxytoca* MK01 alone. Both strains were screened in the SPF ampicillin mouse model. SPF mice were treated with 0.5 g/L ampicillin in their drinking water and pre-colonized with 10⁸ CFUs of the *E. coli* MR102 or PBS, after four days of pre-colonization all mice were challenged with 10⁸ CFUs of the respective MDR *E. coli* strain. Afterwards, fecal colonization levels of MDR *E. coli* were monitored over six weeks, by plating on selective agar plates. CFUs of MDR *E*. *coli* 21236 (ST131) in the group pre-colonized with E. coli MR102 were reduced more than 1000-fold already at day one, from 10¹⁰ CFU/g in PBS treated group to 10⁶ CFU/g. This effect further increased by day six. At day nine, all mice pre-colonized with *E. coli* MR102, reduced the CFU/g of the MDR *E. coli* strain 21236 below the detection limit, while PBS-treated mice were still colonized to 10⁹ CFU/g. Until the end of the experiment, the pre-colonized mice remained without detectable MDR *E. coli* 21236, while PBS-treated mice maintained colonization levels up to 10⁵ CFU/g until day 42 (Figure 4A).

In contrast to MDR *E. coli* strain 21236 (ST131), which was well cleared, strain 55652 (ST167) showed different colonization kinetics throughout the experiment. During ampicillin treatment, the inventors could not observe a reduction of CFUs between the PBS- and *E. coli* MR102-treated groups. Beginning on day six, a 10 to 100-fold reduction of CFU/g was observed between the two groups. Notably, four out of nine mice could even clear out the MDR *E. coli* 55652, whereas the other mice remained colonized to 10⁷-10⁸ CFU/g at the end of the experiment. The PBS-treated group maintained colonization levels of 10⁹-10¹⁰ CFU/g until day 42 (Figure 4B).

Thus, the inventors could demonstrate that the *E. coli* strain MR102 harbors the potential to decolonize different STs of MDR *E*. *coli* strains. In the case of the competition between *E. coli* MR102 and *E. coli* 55652, the inventors observed no competition *ex vivo,* but clearance in 50 % of mice screened *in vivo* (Figure 4B).

### Combination of E. coli MR102 and K. oxytoca MK01 shows the ability to decolonize different strains of MDR Enterobacteriaceae

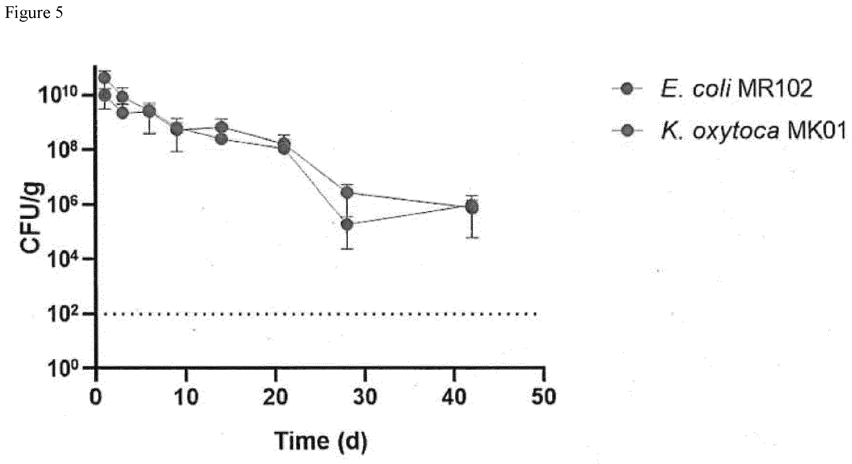

Since the inventors could demonstrate *ex vivo* that a combination of *E. coli* MR102 and *K. oxytoca* MK01 enables competition against a broader spectrum of *E. coli* isolates, the inventors wanted to investigate the decolonization potential of strain combinations in vivo. Since combinations of candidate probiotic strains could also interfere with each other, the inventors first tested whether they could co-colonize SPF mice. Therefore, SPF mice treated with 0.5 g/L ampicillin in their drinking water, were colonized with a 1:1 mixture of *E. coli* MR102 and *K. oxytoca* MK01 (10⁸ CFUs in total). Ampicillin treatment was stopped at day three and fecal colonization of both strains was monitored at different time points for a time course of six weeks. Notably, both strains showed similar colonization levels in the feces at all time points demonstrating that they can co-exist in the gut. During ampicillin treatment on days one and three, colonization levels were at 10¹⁰ CFU/g. After withdrawal of the antibiotic, the colonization levels decreased to 10⁹ CFU/g on day 21 and remained stable at 10⁶ CFU/g until the end of the experiment on day 42 (Figure 5).

Since the inventors could demonstrate that the commensal strains *E. coli* MR102 and *K. oxytoca* MK01 can co-colonize the gut of SPF mice, the inventors selected the MDR *E. coli* strain 55652 (ST167) as a challenge strain, since the inventors observed that this strain cannot be fully cleared by *E. coli* MR102 alone.

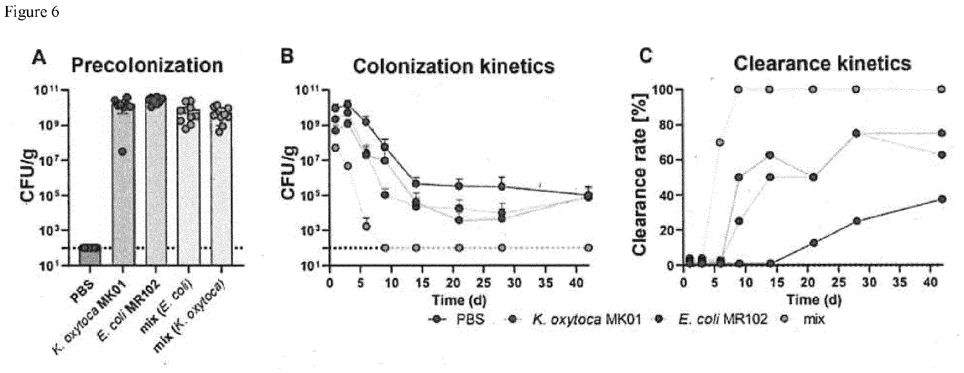

To investigate whether cooperation improves clearance, the inventors compared mice colonized with either *E. coli, K. oxytoca,* or the mixture to control mice. Of note, pre-colonization levels of the two commensal strains were comparable at 10¹⁰ CFU/g, but slightly lower in co-colonized mice (Figure 6A). After a challenge with 10⁸ CFUs of the MDR *E. coli* 55652, CFUs in groups pre-colonized with either *E. coli* MR102 or *K. oxytoca* MK01 were reduced 5 to 10-fold at day one and three compared to the PBS-treated group, whereas the group pre-colonized with the mixture of both strains reduced the CFU/g 1000-fold. This effect further increased throughout the experiment.

While the groups pre-colonized with *K. oxytoca* MK01 or *E. coli* MR102 could only reduce the CFUs of *E. coli* 55652, groups pre-colonized with the combination of both strains could clear out the MDR *E. coli* 55652 to the detection limit by day nine. The CFUs remained stable throughout the rest of the experiment, the PBS-treated group showed 10⁵ CFU/g, and the groups pre-colonized with one of the strains showed 10⁴-10⁵ CFU/g (Figure 6B). Comparing the clearance kinetics, the inventors could observe 100 % clearance in the groups pre-colonized with the mix and 60% clearance for groups pre-colonized with either one of the strains (Figure 6C), demonstrating that the combination of probiotic candidates is more effective in displacing *E. coli* strain 55652 (ST167).

Based on the results that a mixture of *E. coli* MR102 and *K. oxytoca* MK01 can decolonize MDR *E*. *coli* 55652 specifically, the inventors wanted to analyze whether the combination is also able to compete against different MDR-E strains or gastrointestinal pathogens in vivo. The inventors selected a panel of four MDR-E strains comprising a strain of *E. coli* (strain MHH), *E. cloaceae* (strain MHH), *K. pneumoniae* (strain MD), *P. mirabilis* (strain MHH), and the enteric pathogen *S. Typhimurium* (strain EM12442). Of note, it was shown previously that *E. coli* MR102 alone could decolonize the *E. coli* MHH and that *K. pneumoniae* MD can be decolonized by *K. oxytoca* MK01. Thus, for these two instances, it was of great interest to observe if the protective effect will be increased or diminished due to a co-colonization with *E. coli* MR102 and *K. oxytoca* MK01.

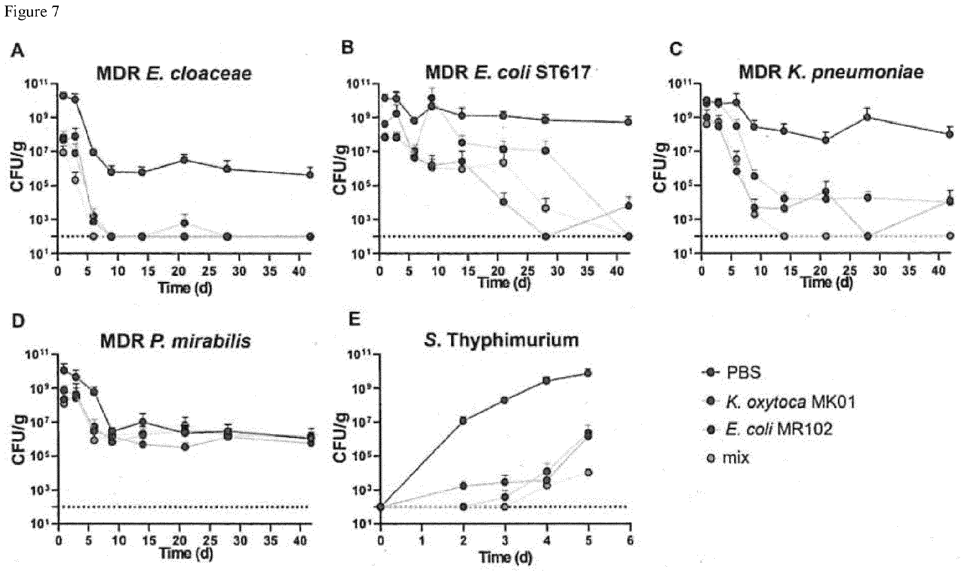

To investigate the competitive effects *in vivo,* mice were pre-colonized with either *E. coli* MR102 or *K. oxytoca* MK01 alone or in combination, and another group was treated with PBS. After a successful pre-colonization, 10⁸ CFUs of the respective MDR-E strain or 10⁵ CFUs of *S. Typhimurium* were administered. CFU/g of the MDR *E. cloaceae* strain could be reduced to the detection limit by day six in mice pre-colonized with the combination. Pre-colonization with one of the two strains alone led to a reduction of CFUs to the detection limit one day later on day nine. Colonization levels in the PBS-treated group remained at 10⁷ CFU/g at day 42 (Figure 7A). *E. coli* MHH and *K. pneumoniae* MD could also be reduced to the detection limit by day 42 (*E. coli* MHH) or day 14 (*K. pneumoniae* MD) (Figure 7B, C). The inventors could not observe a reduction of colonization levels more than 10-fold of the MDR *P. mirabilis* strain in groups pre-colonized with the single strains or the combination (Figure 7D). S. *Typhimurium* colonization levels were strongly reduced after pre-colonization with either *E. coli* MR102 or *K. oxytoca* MK01 and the combination. Five days after the challenge with S. *Typhimurium,* CFU/g in groups pre-colonized with the single strains were reduced 1000-fold, whereas a pre-colonization with the combination led to a 10.000-fold reduction (Figure 7E). Clearance levels of 100 % could be observed for MDR *E. cloaceae, E. coli,* and *K. pneumoniae.*

To summarize, the inventors could demonstrate that a combination of *E. coli* MR102 and *K. oxytoca* MK01 can decolonize a variety of clinically relevant MDR-E strains and gastrointestinal pathogens. By combining the two probiotic strains, the target spectrum was broadened in comparison to the use of one strain by itself, and this indicates a synergistic effect. Further screening is required to validate and expand the target spectrum of the combination. Furthermore, it needs to be investigated how these bacteria enable competition against such a broad spectrum of relevant pathogens.

### References as cited

1. Osbelt, L. et al. Klebsiella oxytoca causes colonization resistance against multidrug-resistant K. pneumoniae in the gut via cooperative carbohydrate competition. Cell Host Microbe 29, 1663-1679.e7 (2021).
2. Eisenhard, L. Investigating the ecological niche of commensal and multidrug-resistant Escherichia coli strains in the gut environment. Tech. Univ. Braunschweig Masterthesis, (2023).
3. Eberl, C. et al. E. coli enhance colonization resistance against Salmonella Typhimurium by competing for galactitol, a context-dependent limiting carbon source. Cell Host Microbe 29, 1680-1692.e7 (2021).
4. Oliveira, R. A. et al. Klebsiella michiganensis transmission enhances resistance to Enterobacteriaceae gut invasion by nutrition competition. Nat. Microbiol. 5, 630-641 (2020).
5. Banerjee, R. & Johnson, J. R. A new clone sweeps clean: The enigmatic emergence of Escherichia coli sequence type 131. Antimicrob. Agents Chemother. 58, 4997-5004 (2014).
6. Johnson, J. R., Johnston, B., Clabots, C., Kuskowski, M. A. & Castanheira, M. Escherichia coli sequence type ST131 as the major cause of serious multi drug-resistant E. coli infections in the United States. Clin. Infect. Dis. 51, 286-294 (2010).

## Claims

1. A bacterium selected from the group of consisting of at least one of *E. coli* strains MR010, MK031, MR043, MR045, MR102, MR134, MR158, MK241, MK249, MK267, MK270, MK289, MR107, MR050, MK254, MR174, MR084, MK251, MK062, MR097, MK270, MR138, MK262, and MK179 for use in the prevention and/or treatment of pathogenic and/or antibiotic resistant *Enterobacteriaceae,* such as *E. coli* and/or *Klebsiella pneumoniae,* in particular multi-resistant (MDR) *Enterobacteriaceae, Enterococci* and/or *Acinetobacter* in the microbiome of an animal, in particular an avian or mammalian subject.

2. Bacterium for use according to claim 1, wherein said use is in combination with at least one bacterium selected from the group of *Klebsiella michiganensis and*/*or Klebsiella oxytoca,* in particular from the strain MK01, MR08, MR050, and species related to *Klebsiella oxytoca* MK01, and wherein preferably the combination shows a synergistic effect in the prevention and/or treatment.

3. Bacterium for use according to claim 2, wherein the combination is selected from the group of *E. coli* strains MR102, and MK289 with *Klebsiella oxytoca* MK01, MR08, or *Klebsiella michiganensis* MR050, preferably MK289 with *Klebsiella oxytoca* MK01.

4. Bacterium for use according to any one of claims 1 to 3, wherein said pathogenic and/or antibiotic resistant *Enterobacteriaceae* is selected from at least one of *E. coli* strains MHH, 56922, 56236, 56589, 56231, 55652, 55838, 54519, 55476, 56035, 21236, 26260, 53221, 54875, 44583, 51583, PBIO729, PBIO730, and EPEC, preferably MHH, 56922, 56231, 21236, 53221, PBIO729, and EPEC.

5. Bacterium for use according to any one of claims 2 to 4, wherein said species related to *Klebsiella oxytoca* is selected from the group consisting of *K. michiganensis,* and *Klebsiella* that are related to or have a carbohydrate utilization pattern substantially identical to *K. oxytoca* strain MK01, MR08, or *Klebsiella michiganensis* MR050.

6. Bacterium for use according to any one of claims 1 to 5 in reestablishing colonization resistance after treatment and/or antibiotic dysbiosis in the microbiome of an animal, in particular an avian or mammalian subject.

7. Bacterium for use according to any one of claims 1 to 6, wherein said microbiome is located in the gut of said animal, in particular said an avian or mammalian subject.

8. Bacterium for use according to any one of claims 1 to 7, wherein said prevention and/or treatment of said pathogenic and/or antibiotic-resistant *Enterobacteriaceae* and/or said reestablishing colonization resistance after treatment and/or antibiotic dysbiosis is in the context of bacterial infection, inflammatory bowel disease (IBD), bloodstream infection, sepsis, allogeneic hematopoietic stem cell transplantation, and bacterial invasion into the liver, spleen and/or mesenteric lymph nodes (MLN) in said animal, in particular said avian or mammalian subject.

9. Bacterium for use according to any one of claims 1 to 9, wherein said use comprises fecal microbiota transplant (FMT), in a suitable medium, such as sterile saline, or in a suitable solid dosage form, such as a suppository or capsule, in particular stomach-acid resistant, or as a probiotic.

10. Bacterium for use according to any one of claims 1 to 10, wherein said use is in a dosage of between 10¹² and 10⁷ CFU, preferably between 10¹⁰ and 10⁸ CFU of said bacterium.

11. Bacterium for use according to any one of claims 1 to 10, wherein said use is in combination with at least one bacterial species selected from the group consisting of Bacteroidetes, Actinobacteria, and Firmicutes spec., and preferably from the families Lachnospiraceae, such as, for example, Schaedlerella, Chodladocola, Acutalibacteraceae, such as for example Eubacterium_R, Lactobacillaceae, such as, for example, Ligilactobacillus, Eggerthellaceae, such as, for example, Adlercreutzia, and Rikenelleaceae, such as, for example, Alistipes, and in particular human isolates thereof.
